Europäisches Patentamt

European Patent Office

Office européen des brevets

(11) Veröffentlichungsnummer: **0 031 018 B1**

(12) **EUROPÄISCHE PATENTSCHRIFT**

(45) Veröffentlichungstag der Patentschrift:
22.02.84

(51) Int. Cl.³: **A 61 L 15/01,** A 61 L 15/00, A 61 F 13/00

(21) Anmeldenummer: **80106883.4**

(22) Anmeldetag: **08.11.80**

(54) Abdeckvorrichtung zur Behandlung der Haut auf Basis von gelartigen Polymerisaten.

(30) Priorität: **17.11.79 DE 2946553**

(43) Veröffentlichungstag der Anmeldung:
**01.07.81 Patentblatt 81/26**

(45) Bekanntmachung des Hinweises auf die Patenterteilung:
**22.02.84 Patentblatt 84/8**

(84) Benannte Vertragsstaaten:
**AT BE CH DE FR GB IT LI NL SE**

(56) Entgegenhaltungen:
**CH - A - 316 547
CH - A - 485 861
CH - A - 488 458
DE - B - 1 190 608
FR - A - 1 534 771
FR - A - 2 122 432
FR - A - 2 181 756
US - A - 3 062 209
US - A - 3 674 901
US - A - 3 890 974**

(73) Patentinhaber: **HOECHST AKTIENGESELLSCHAFT,
Postfach 80 03 20, D-6230 Frankfurt am Main 80 (DE)**

(72) Erfinder: **Walch, Axel, Dr., Hans-Sachs-Strasse 5,
D-6000 Frankfurt/Main 90 (DE)**

## Abdeckvorrichtung zur Behandlung der Haut auf Basis von gelartigen Polymerisaten

Die Erfindung bezieht sich auf eine Abdeckvorrichtung zur Behandlung der Haut, insbesondere bei Verbrennung, Wunden und Hautdefekten, bestehend aus einer selektiv durchlässigen Membranschicht mit an dieser angrenzendem, Wasser enthaltendem Gel aus Polymerisat.

Für die Therapie großflächiger Wunden, beispielsweise bei schweren Verbrennungen, werden von dem Abdeckmaterial äußerst vielfältige, zum Teil gegensätzliche Eigenschaften gefordert, welche die bekannten Vorrichtungen bisher nur unzureichend besitzen. So soll das Abdeckmaterial einerseits zur Aufnahme von Wundsekret geeignet sein, andererseits aber Wasser- oder Proteinverluste des Patienten möglichst gering halten. Es soll Wärme- und Gasaustausch ermöglichen, aber den Zutritt von Bakterien und Viren verhindern. Es soll möglichst schmerzfrei gewechselt werden können, jedoch die Granulation der Haut durch ein feuchtes Medium stimulieren und durch Wasserzufuhr eine Verdünnung von Sekret und Toxinen ermöglichen. Ferner wird verlangt, daß Medikamente epikutan appliziert werden können.

Deshalb werden in der Praxis zur Wundbehandlung, beispielsweise bei schweren Verbrennungen im allgemeinen natürliche Materialien, wie Schweinehaut oder autologe Transplantate, eingesetzt, allerdings erst nach Entfernen des nekrotischen Gewebes. Transplantate sind zwar Schweinehaut vorzuziehen, jedoch sind sie ebenfalls in der ersten Phase der Wundsanitation, nämlich zur Reinigung der Wunde, nicht anwendbar. Außerdem stehen sie gewöhnlich nicht in der erforderlichen Menge zur Verfügung.

In der CH-A-488 458 wird ein Wundverschlußmittel beschrieben, welches einlagig ausgebildet und biegsam ist. Es besitzt Eigenformstabilität, ist aber an die Kontur des verletzten Bereiches anpaßbar. Die das bekannte Wundverschlußmittel bildende, selbständig formstabile Schicht besteht aus vernetztem Polymeren bestimmten Aufbaus, welches sich im Gelzustand befindet, es besitzt aber nicht so hohe Feuchtigkeitswerte, daß es insbesondere bei schweren Verbrennungen die Wunde wirksam und nachhaltig vor dem Austrocknen bewahren kann.

In der FR-A-2 181 756 wird künstlich Haut beschrieben, die zweischichtig ist. Sie besteht aus einer Schwammschicht aus hydrophilem Polymerisat, die Wasser enthält und die mit einer dünnen Schicht eines Films aus hydrophobem Polymerisat verbunden ist. Der Film ist so ausgebildet, daß er feuchtigkeits-, dampf-, sauerstoff- sowie kohlendioxiddurchlässig ist. Auch dieses Schichtmaterial trocknet an der Luft leicht aus und kann vor allem größere Wunden dann nicht mehr wirksam schützen.

Es ist auch bekannt, vernetzte, gelartige Polymerisate, d. h. wasserhaltige Polymerisatmassen, wie z. B. vernetztes Polyacrylamid-Gel oder Agar-Gel, zur Wundbehandlung und in der Dermatologie einzusetzen. Diese Polymerisatmassen lassen sich jedoch nur sehr schwer auf kontinuierliche Weise in Form von Schichten oder folienartigen Gebilden herstellen und zeigen außerdem schwerwiegende Mängel im klinischen Einsatz, da sie nur umständlich handhabbar sind.

Es ist deshalb Aufgabe der Erfindung, ein weiteres, verbessertes Mittel auf Basis von Polymeren-Gel zur Behandlung der Haut, insbesondere zur Behandlung von Verbrennungen, Wunden, aber auch von Hautdefekten und dermatologischen Problemen, vorzuschlagen. Es soll dem Fachmann die Möglichkeit geben, bestimmte Hautschäden zu behandeln, für die die bisherigen Abdeckvorrichtungen weniger gut geeignet sind.

Diese Aufgabe wird gelöst durch eine Abdeckvorrichtung zur Behandlung der Haut, insbesondere zur Behandlung von Hautverbrennungen, Wunden sowie Hautdefekten, bestehend aus einer selektiv durchlässigen Membranschicht mit an dieser angrenzendem, Wasser enthaltendem Gel aus Polymerisat, dadurch gekennzeichnet, daß sie aus einem wasserdampf- sowie gasdurchlässigen, flachen, an seinen Enden abgedichteten Membranschlauch mit Poren einer durchschnittlichen Größe von $3 \times 10^{-4}$ bis 10 µm besteht, dessen Hohlraum leicht deformierbare sowie leicht fließfähige Masse aus Polymeren-Gel mit in bezug auf dessen Gesamtgewicht hohem Wasseranteil enthält.

Weitere Ausführungsformen der Vorrichtung sind in den Unteransprüchen 2 bis 4 genannt und untenstehend beschrieben.

Der Membranschlauch, in dessen Hohlraum sich die leicht deformierbare sowie leicht fließfähige Masse aus Polymeren-Gel befindet, und welcher wasserdampf- und gasdurchlässig ist, ist insbesondere aus einer Membran gebildet, mit einer durchschnittlichen Porengröße im Bereich von $3 \times 10^{-4}$ bis 10 µm, und besteht beispielsweise aus Celluloseregenerat (DE-AS 2 736 569) oder einer asymmetrischen Kunststoffmembran aus Polyamid (DE-AS 2 751 910) oder Polycarbonat (DE-OS 2 510 337). Diese Membranen wurden bisher bei der Dialyse (Porengröße $10^{-3}$ bis $10^{-2}$ µm) oder Hämofiltration verwendet und zeigen eine Dicke von 10 bis 150 µm. Die Membran besitzt die Form eines flachgelegten Schlauches, der an seinen Enden abgedichtet oder verschlossen ist, z. B. in Form eines Beutels, der das gesamte Polymeren-Gel umgibt.

Das Polymeren-Gel ist insbesondere leicht deformierbar bzw. fließfähig, im Gegensatz zu den bisher zur Wundbehandlung eingesetzten vernetzten Polymerisaten. Sein nicht aus Wasser bestehender Anteil besteht z. B. aus einem Cellulose- oder Stärkeether, insbesondere aus Hydroxyethylcellulose, Carboxymethylcellulose oder Methylcellulose oder dementsprechendem Stärkederivat. Geeignete Celluloseether sind ferner Alkylhydroxyalkylether, Alkylcarboxymethylether, Hydroxyalkylcarboxymethylether und Alkylhydroxyalkylcarboxymethylether.

Weiterhin sind geeignete Polymeren-Gele z. B. Gele auf Basis von Acrylsäure und Methacrylsäure oder deren Derivaten sowie Polyvinylpyrrolidon, Polyvinylalkohol, Agar oder Agarose.

Es ist aber auch möglich, chemisch modifizierte Celluloseether zu verwenden, wie sie in den DE-OS 1 912 740, 2 357 079, 2 358 150 und 2 519 927 beschrieben werden.

Auch mit ein-, zwei- oder dreiwertige Ionen enthaltenden Verbindungen modifizierte Celluloseether sind geeignete Polymeren-Gele.

Die Schichtdicke des Polymeren-Gels kann je nach Verwendungszweck in weiten Grenzen variieren, liegt jedoch zweckmäßigerweise bei etwa 1 bis 5 mm. Bei Verwendung einer Vorrichtung mit einem transparenten Polymeren-Gel und einer transparenten Membran zur Behandlung von Hautschäden läßt sich der Heilvorgang vorteilhafterweise optisch verfolgen und kontrollieren.

In weiterer Ausführungsform steht die Membran mit einem Reservoir für Medikamente in Verbindung. Dieses Reservoir wird z. B. durch eine flüssigkeitsdichte Folie gebildet, welche die Membran auf der von der zu behandelnden Haut abgewandten Seite abdeckt und mit dieser einen Hohlraum bildet. Die Oberfläche der Membran, welche an diesen Hohlraum angrenzt, ist gegebenenfalls mit einer flüssigkeitsdichten Folie mit Öffnungen oder Sollbruchstellen abgedeckt, welche erst unmittelbar vor der Verwendung der Abdeckvorrichtung geöffnet werden. In diesen Hohlraum wird beispielsweise eine Bakterien tötende oder therapeutisch wirksame Lösung eingefüllt, die durch die Membran in das Polymeren-Gel gelangt und dann auf die zu behandelnde Hautstelle auftrifft. Dieses Reservoir ermöglicht somit eine rasche Befüllung und/oder eine kontinuierliche Nachdosierung von Flüssigkeit und Medikamenten in die Abdeckvorrichtung. Dabei wird die Dosierungsgeschwindigkeit von der Art und Porengröße der Membran gesteuert. Es ist auch möglich, das Polymeren-Gel bereits beim Einfüllen in einen Membranschlauch mit der therapeutisch wirksamen Lösung zu vermischen.

Die Abdeckvorrichtung der Erfindung erfüllt die eingangs genannten Aufgaben. Sie läßt sich ferner auf wirtschaftliche Weise fertigen und ist im klinischen Einsatz einfach zu handhaben.

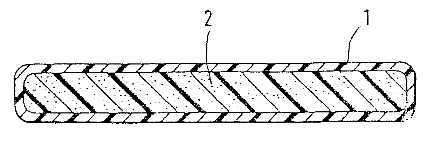

In der Figur wird eine Ausführungsform im Schnitt gezeigt. Sie besteht aus einer Membran in Form eines Schlauchbeutels 1, in dessen Hohlraum sich die leicht deformierbare sowie leicht fließfähige Masse aus Polymeren-Gel 2 befindet.

## Patentansprüche

1. Abdeckvorrichtung zur Behandlung der Haut, insbesondere zur Behandlung von Hautverbrennungen, Wunden sowie Hautdefekten, bestehend aus einer selektiv durchlässigen Membranschicht mit an dieser angrenzendem, Wasser enthaltendem Gel aus Polymerisat, dadurch gekennzeichnet, daß sie aus einem wasserdampf- sowie gasdurchlässigen, flachen, an seinen Enden abgedichteten Membranschlauch mit Poren einer durchschnittlichen Größe von $3 \times 10^{-4}$ bis 10 µm besteht, dessen Hohlraum leicht deformierbare sowie leicht fließfähige Masse aus Polymeren-Gel mit in bezug auf dessen Gesamtgewicht hohem Wasseranteil enthält.

2. Abdeckvorrichtung nach Anspruch 1, dadurch gekennzeichnet, daß der verschlossene Membranschlauch als Schlauchbeutel ausgebildet ist.

3. Abdeckvorrichtung nach Anspruch 1 und 2 dadurch gekennzeichnet, daß Schlauch- sowie Polymeren-Gel-Masse jeweils transparent sind.

4. Abdeckvorrichtung nach Anspruch 1 bis 3, dadurch gekennzeichnet, daß die leicht deformierbare sowie leicht fließfähige Masse aus Polymeren-Gel als Polymerisate solche enthält, die ausgewählt sind aus einer Gruppe umfassend Kohlehydrate, Kohlehydratderivate, Polyvinylpyrrolidon, Polyvinylalkohol, Polyacrylsäure, Derivate der Polyacrylsäure, Polymethacrylsäure sowie Derivate der Polymethacrylsäure.

## Claims

1. Covering device for skin treatment, in particular for the treatment of burns, wounds and skin lesions, comprising a selectively permeable membrane layer with an adjoining watercontaining gel formed from a polymer, wherein a flat membrane tube which is permeable to water vapour and gases and is sealed at its ends and has pores an average size from $3 \times 10^{-4}$ to 10 µm, contains in its cavity a readily deformable and readily flowable mass of a polymer gel, which has a high content of water relative to its total weight.

2. A covering device as claimed in claim 1, wherein the closed membrane tube has the shape of a tubular bag.

3. A covering device as claimed in claims 1 and 2, wherein the material forming the tube and also the mass of polymer gel are transparent.

4. A covering device as claimed in claims 1 to 3, wherein the readily deformable and readily flowable mass of polymer gel comprises polymers of a kind selected from a group consisting of carbohydrates, carbohydrate derivatives, polyvinylpyrrolidone, polyvinyl alcohol,

polyacrylic acid, derivatives of polyacrylic acid, polymethacrylic acid and derivatives of polymethacrylic acid.

## Revendications

1. Dispositif de recouvrement pour le traitement de la peau, en particulier pour le traitement des brûlures de la peau, des plaies ainsi que des défauts de la peau, composé d'une membrane à perméabilité sélective et d'un gel à base de polymérisat contenant de l'eau et adjacent à cette membrane, caractérisé en ce qu'il est composé d'un tube formé d'une membrane, perméable à l'eau, à la vapeur ainsi qu'aux gaz, plat, fermé à ses extrémités, présentant des pores d'un dimension moyenne de $3 \times 10^{-4}$ à 10 μm, dont la cavité contient une masse facilement déformable ainsi que facilement apte à couler, formée d'un gel de polymère comportant une teneur élevée en eau comparativement à son poids total.

2. Dispositif de recouvrement selon la revendication 1, caractérisé en ce que le tube fermé est réalisé sous la forme d'un sachet tubulaire.

3. Dispositif de recouvrement selon les revendications 1 et 2, caractérisé en ce que le tube ainsi que la masse de gel de polymère sont transparents.

4. Dispositif de recouvrement selon les revendications 1 à 3, caractérisé en ce que la masse facilement déformable et facilement apte à couler de gel de polymère contient, comme polymérisats, des polymérisats qui sont choisis parmi les hydrates de carbone, les dérivés d'hyrates de carbone, la polyvinylpyrrolidone, l'alcool polyvinylique, l'acide polyacrylique, les dérivés de l'acide polyacrylique, l'acide polyméthacrylique et les dérivés de l'acide polyméthacrylique.